# EUROPEAN PATENT SPECIFICATION

(11) **EP 2 320 621 B1**
(45) Date of publication and mention of the grant of the patent: **05.10.2016**
(21) Application number: 10013409.7
(22) Date of filing: 07.10.2010
(51) Int. Cl.: H04L 29/06, H04L 9/08, G06F 19/00, G06F 21/60, H04L 9/32, H04L 9/00, H04L 9/30

(54) **Method for establishing cryptographic communications between a remote device and a medical device and system for carrying out the method**
Verfahren zur Herstellung von kryptografischen Kommunikationen zwischen einer Ferneinrichtung und einer medizinischen Vorrichtung und System zur Durchführung des Verfahrens
Procédé pour établir des communications cryptographiques entre un dispositif distant et un dispositif médical et système permettant d'effectuer le procédé

(30) Priority: 06.11.2009 EP 09013940
(43) Date of publication of application: 11.05.2011
(73) Proprietor: F.Hoffmann-La Roche AG, 4070 Basel (CH); Roche Diabetes Care GmbH, 68305 Mannheim (DE)
(72) Inventor: Konrad, Guido, 3006 Bern (CH); Trösch, Martin, 4900 Langenthal (CH); Linder, Felix, 10117 Berlin (DE); Kopf, Gregor, 10589 Berlin (DE)
(74) Representative: Rentsch Partner AG

(56) References cited:
- EP-A1- 1 770 900
- EP-A1- 1 973 265
- WO-A1-2004/017600
- WO-A1-2008/154467
- GB-A- 2 404 126
- US-A1- 2002 087 884
- US-A1- 2003 065 918
- US-B1- 6 233 617
- MCCUNE J M ET AL: "Seeing-Is-Believing: Using Camera Phones for Human-Verifiable Authentication", SECURITY AND PRIVACY, 2005 IEEE SYMPOSIUM ON OAKLAND, CA, USA 08-11 MAY 2005, PISCATAWAY, NJ, USA,IEEE, 8 May 2005 (2005-05-08), pages 110-124, XP010798367, DOI: 10.1109/SP.2005.19 ISBN: 978-0-7695-2339-2

## Description

The invention relates to a method for establishing cryptographic communications between a remote device and a medical device according to the preamble of claim 1 and to a system with a remote device and a medical device according to the preamble of claim 19. Both the remote device and the medical device are electronic devices with some processing power.

As medical device and remote device may be employed, for example an infusion pump, and a diabetes management device, wherein the infusion pump constitutes the medical device and the diabetes management device constitutes the remote device. The remote device may also be, for example, a communication device, a blood glucose meter, a smart phone, a personal digital assistant (PDA), a personal computer (PC), or a remote terminal representing a remote infusion pump display on which data from the medical device is displayed to a user. The remote device may also include a bolus advisor by which the user can command the administration of insulin through the infusion pump and on which the delivery history of the infusion pump is displayed. Other types of medical devices and remote devices are possible.

From WO 2008/154467 A1 is known a method for pairing and authenticating a medical device with a remote electronic device, wherein Bluetooth pairing is used for the pairing. For authenticating an e.g. 10 digit PIN code that has been generated by the medical device has to be entered by the user into the remote device. A PIN code with more or less digits, for example an 8 digit PIN code may be employed depending on practical considerations. Then the medical device and the remote device generate each a signature from the PIN code. The remote device sends its signature to the medical device and compares the signature generated by the remote device to the signature generated by the medical device. If the signatures are the same, authentication has been successful. Manually entering a PIN code imposes a certain burden onto the user. Furthermore, for the PIN code to be a secure PIN code it usually has to be rather large, i.e. up to 40 digits, which makes entering the PIN code even more inconvenient for the user and which can be rather difficult with limited displays and user interfaces.

A more secure key exchange can be obtained by public-key cryptography that uses an asymmetric-key method. With an asymmetric-key method the key used to encrypt a message is not the same as the key used to decrypt it. Each user has a pair of cryptographic keys - a public key and a private key. The private key is kept secret, whilst the public key may be widely distributed. Messages are encrypted with the recipient's public key and can only be decrypted with the corresponding private key. The keys are related mathematically, but the private key can typically not be feasibly derived from the public key (confer http://en.wikipedia.org/wiki/Public-key_cryptography). Examples for asymmetric-key methods are the Diffie-Hellman key exchange, the more secure and faster RSA communications method that is suitable for signing as well as for encryption (confer http://en.wikipedia.org/wiki/Rsa, US 4,405,829), Transport Layer Security (TLS) that provides security for communications over networks such as the Internet but requires digital certificates for the key exchange, the Rabin and Elgamal cryptosystems, and cryptosystems based on elliptical curves.

While public-key methods have the advantages that sender and recipient do not have to share a common secret (a shared secret key, with one copy of the secret key at the sender's side and one copy at the recipient's side) in order to communicate securely and that a secure initial exchange of a secret key is not required in contrast to symmetric-key methods (confer http://en.wikipedia.org/wiki/Symmetric_key_algorithm), they often rely on complicated mathematical computations and, hence, require much higher processing power than a symmetric-key method or run much slower on systems with the comparable processing power, respectively. Symmetric-key cryptosystems are generally much less computationally intensive and more efficient than comparable public-key cryptosystems. In practice, asymmetric-key methods are typically hundreds to thousands times slower than comparable symmetric-key methods. Therefore a key exchange with conventional public-key cryptography is often problematic with existing battery-driven devices, such as e.g. embedded devices (for example an insulin pump), due to their limited processing power and hence limited clock rate and their limited memory capacity. With the known asymmetric-key methods, in particular with the RSA communications method, the encryption is significantly faster than the decryption, i.e. significantly more processing power is required for the decryption.

WO 2004/017600 relates to encryption and data security for devices with limited resources. A first device sends a public encryption key together with acceleration data to a second device. The second device uses the acceleration data and the public key to generate an encrypted private session key, which is sent back to the first device. The encrypted private session key is decrypted into the private session key using the private encryption key. The private session key is used to initialize a private-key encryption method.

GB 2 404 126 relates to secure communications links in which asymmetric cryptographic techniques are used to establish a secure link using symmetric cryptography. A message including a secret number is digitally signed using a private key of the sender. The message is transmitted to a recipient. The secret number is used for symmetric cryptography and valid only for a certain time period. The message includes a time stamp which is used by the recipient to verify that the secret number is still valid. The secret number is extracted by the recipient using the sender's public key.

EP 1 770 900 relates to securely sharing a session key between a communication apparatus and a communication partner, even if the communication apparatus has a processing power that is not so high. A short-range communication unit transmits an inquiry signal to a short-range external communication apparatus by electromagnetic waves and waits for a response to the inquiry signal. A long-range communication unit is capable of communication by the electromagnetic waves in a wider area than the short-range communication unit. A switching unit switches either to the short-range or the long-range communication unit. An asymmetric key generator generates a pair of encryption key and decryption key. The decryption key is used to decrypt an encrypted session key transmitted from the external communication apparatus. The switching to the long-range communication unit and communication by using the long-range communication unit is requested.

EP 1 973 265 describes a key handling in mobile communication systems where first and second numbers are exchanged between entities of the system. The first and second numbers are respectively used only once with respect to the respective system parameters. Several keys are derived from a semi-permanent key shared between a user equipment and a mobility management entity. Nonces are transmitted in a state transition in order to derive new temporary keys from the semi-permanent key or another temporary key. Nonces include random values, time-stamps, or combination of these.

It is an object of the claimed invention to provide a method for establishing cryptographic communications between a remote device and a medical device, the medical device having less, in particular significantly less, processing power than the remote device, by which method secure communications between a medical device and a remote device can be ensured. By a secure communication it is meant that the communicated data are protected against unauthorized access, use, disclosure, disruption, modification, and destruction and similar. It is a further object of the claimed invention to provide a method for establishing cryptographic communications between a remote device and a medical device, the medical device having less, in particular significantly less, processing power than the remote device, by which method secure communications between a medical device and a remote device can be established and whose security corresponds to the security that can be achieved with public-key algorithms while requiring less processing power. It is a still further object of the invention to provide a system for carrying out the invention.

In order to implement these and still further objects of the invention, which will become more readily apparent as the description proceeds, a method for establishing cryptographic communications between a remote device and a medical device, in particular an insulin pump, is provided, the medical device having less, in particular significantly less, processing power than the remote device. The method according to the invention comprises the steps of establishing unencrypted communication between the remote device and the medical device, generating an asymmetric key pair by the remote device with the asymmetric key pair comprising a public key and a private key, generating a key request message by the remote device and sending the key request message together with the public key to the medical device, generating a pre-master key by the medical device and encrypting the pre-master key with the received public key by the medical device, generating a key response message by the medical device and sending of the key response message together with the encrypted pre-master key from the medical device to the remote device, decrypting the encrypted pre-master key with the private key by the remote device, and deriving a master key as symmetric key from the pre-master key by either using the pre-master key as master key (i.e. the master key corresponds to/is identical with the pre-master key) or by using the key request message and/or the key response message by each of the medical device and the remote device for the derivation of the master key from the pre-master key, the master key to be used for decryption and encryption of application data to be communicated between the medical device and the remote device. Besides decryption and encryption the master key may optionally also be used for authentication purposes.

Regarding the first established unencrypted communication between the remote device and the medical device, it is noted that for this so-called unencrypted communication a communication protocol may be used for the communication between the medical device and the remote device that may comprise its own additional and self-contained encryption, as being e.g. the case with Bluetooth. This additional, self-contained encryption during the first communication establishing step is, however, not considered an encryption in the sense of the present invention.

The generated master key can be used to encrypt and decrypt application data to be communicated according to a symmetric cryptography method based on e.g. AES (Advanced Encryption Standard), DES (Data Encryption Standard), IDEA (International Data Encryption Algorithm), Blowfish, Twofish and/or similar.

The method according to the invention preferably employs the RSA communications method, such that the asymmetric key pair generated by the remote device is an RSA key pair, or another asymmetric-key communications method where encryption is faster, preferably significantly faster, than decryption for generation of the asymmetric key pair, for encryption of the pre-master key and for its decryption.

The remote device acts as client, while the medical device acts as server. A so-called key request/response message is defined as a message sent to announce the presence of a device, i.e. with the key request message the remote device announces its presence to the medical device, and with the key response message the medical device announces its presence to the remote device.

The method according to the invention is unsymmetrical in that encryption of the pre-master key, which requires significantly less processing power than its decryption, takes place on another device, namely the medical device, than the decryption which takes place on the remote device. Hence, the method according to the invention is particularly suitable for set-ups of two devices, wherein (only) one of the devices (here: the medical device) has low processing power and/or low memory, this typically being the case for embedded/implanted devices and mobile battery-driven devices. The computationally expensive decryption is left to the computationally stronger device (here: the remote device) and the medical device with limited processing power only has to perform the encryption. Furthermore, the method according to the invention makes manually entering a PIN code, as required by the state of the art in WO 2008/154467 A1, redundant.

The method according to the invention combines the convenience of public-key cryptography (using an asymmetric key pair) with the efficiency of symmetric-key cryptography (with the master key) and thus constitutes a so-called hybrid cryptosystem. In the method according to the invention public-key cryptography is employed until the pre-master key, which has been encrypted with the public key by the medical device, has been sent to and decrypted by the remote device with the private key to ensure secure communication of the pre-master key. From the pre-master key is derived a master key to increase security, the master key serving as symmetric key. The master key is then used within a more efficient symmetric-key cryptosystem for encryption and decryption and/or authentication of the application data that shall be communicated between the remote device and the medical device.

Under the term "encryption" is understood the process of transforming data/information to make it unreadable to anyone except those possessing special knowledge, i.e. the key, for decrypting the data/information, thereby reversing the process of encryption to make the data/information readable again. Under the term "authenticating" is understood the process of verifying the identity of a device (or a person or an application, respectively).

The method according to the invention may further comprise a verification step, the verification step comprising that the remote device and the medical device each compute verification data, wherein the master key, preferably the pre-master key and/or preferably the previously exchanged key request and key response messages, that preferentially comprise pseudo-random data, are used for the computation of the verification data. The remote device and the medical device each display the verification data to the user, who is prompted to confirm that the verification data computed by the remote device and the verification data computed by the medical device have in fact the same values, i.e. are identical. First after the user has confirmed that the verification data computed by the remote device and the verification data computed by the medical device are identical, cryptographically secured communication of application data between the remote device and the medical device may be started. By providing the verification step so-called man-in-the-middle attacks can advantageously be prevented.

In cryptography, a man-in-the-middle attack (short: MITM; also called bucket-brigade attack or Janus attack) is a form of active eavesdropping, in which the attacker makes independent connections with the victims and relays messages between them, while making them believe that they are talking directly to each other over a private connection when in fact the entire conversation is controlled by the attacker. For a successful man-in-the-middle attack the attacker must be able to intercept all messages going between the two victims and to inject new messages, i.e. the attacker has to be able to impersonate each communication endpoint to the satisfaction of the other (confer http://en.wikipedia.org/wiki/Man-in-the-middle_attack). Through the verification step it can advantageously be ensured that messages sent from the remote device to the medical device and vice versa (such as the key request message together with the public key, or the key response message together with the encrypted pre-master key) have not been manipulated by an attacker.

The verification data as such are preferably binary data. However, in order to allow easy comparison and verification of the verification data displayed by the remote device and the medical device by the user, the verification data may be transformed into a more convenient format, such as an alphanumeric string, by the remote device and the medical device, respectively, for example by employing so-called Base64 character encoding that transforms the binary verification data into printable ASCII characters.

For verifying that the verification data computed by the remote device and the verification data computed by the medical device are identical, various alternative methods may be used, wherein the independent computation of the respective verification data is as described above. Instead of (or for safety reasons additionally to) prompting the user to confirm the identity of the verification data displayed on the remote device with the verification data displayed on the medical device and wait for the user's manual confirmation of the identity, the verification data of one of the devices may be transmitted to the other device e.g. via an additional wireless communication interface such as an IR (infrared) interface that in particular allows transmission via short transmission ranges of less than 1 metre, particularly less than 50 centimetres. Such an IR interface may already have been provided by the device's manufacturer for configuration and service purposes. Alternatively, the verification data may be displayed by one of the devices (either e.g. as alphanumeric string or as pixel-graphics) and the other device may comprise a camera for recording the displayed verification data. Still alternatively, one of the devices may transform the computed verification data into an audible signal that is received by a corresponding receiver e.g. a microphone of the other device. The comparison of the two sets of verification data is then automatically performed by the one of the devices that receives the verification data from the other device. For all of the above-described alternatives for performing the verification step both the medical device and the remote device should be placed in - preferably close - proximity to each other.

The invention further relates to a system comprising of a remote device and a medical device, in particular an insulin pump, with the medical device having less processing power than the remote device, wherein the system is designed and/or configured to carry out the method of the invention.

Further advantageous features and applications of the invention can be found in the dependent claims as well as in the following description of the drawings illustrating the invention. In the drawings like reference numerals designate the same or similar parts or method steps throughout the several figures of which:
Figure 1 shows an abstract interconnection layer model for wireless communications between a medical device and a remote device,
Figure 2 shows a flowchart of a first embodiment of the method of the invention,
Figure 3 shows a flowchart of a resumption of a session which has been started in accordance with the first embodiment shown in Figure 2, and
Figure 4 shows a flowchart of a second embodiment of the method of the invention.

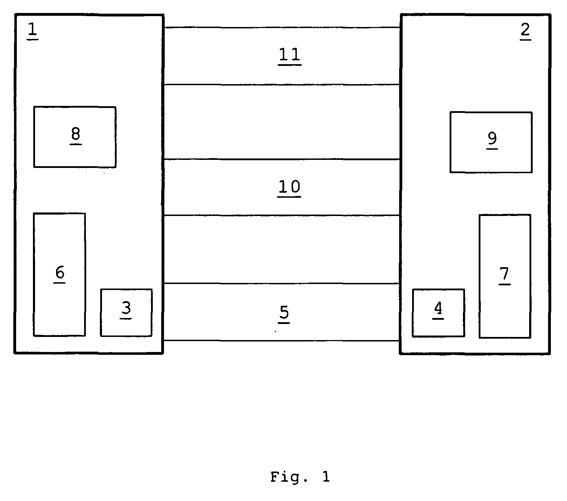

Figure 1 shows a medical device 1 and a remote device 2 along with an abstract interconnection layer model for wireless communication between the two devices 1 and 2. The medical device 1 may in particular be a mobile medical device such as an insulin pump. The remote device 2 may for example be a glucose meter or a personal computer. The medical device 1 includes a wireless communication module 3 and the remote device 2 also includes a wireless communication module 4. The wireless communication modules 3 and 4 communicate with each other wirelessly via a communications layer 5. The wireless communication modules 3 and 4 each include a dedicated processor connected to a wireless transmitter and a wireless receiver, or to a wireless transceiver. The wireless communication modules 3 and 4 are configured to communicate with each other using any conventional wireless medium including, for example, radio frequency (RF), infrared (IR), microwave, inductive coupling, or the like. The communications layer 5 supports the chosen wireless medium. The communication modules 3 and 4 may, for example, each be configured to communicate via RF according to a conventional BlueTooth® radio frequency communications protocol, and the communications layer 5 may accordingly be a conventional BlueTooth® communications layer.

The medical device 1 and the remote device 2 each further include a control unit 6 and 7 respectively. The control units 6 and 7 may be or include a microprocessor, although either or both of the control units 6 and 7 may additionally or alternatively include other control circuits. The medical device 1 and the remote device 2 each further include a user interface 8 and 9 respectively. The user interfaces 8 and 9 may be visual display units, each of which may be or include a conventional liquid crystal display (LCD), one or more light emitting diodes (LEDs), a vacuum fluorescent display or the like. The user interfaces 8 and 9 may also comprise either or both an audible device configured to be responsive to a control signal to produce an audible sound and a tactile device configured to be responsive to a control signal to produce a tactile signal that may be perceived, i.e. felt, by a user. The user interfaces 8 and 9 may also comprise some input unit, such as keys, a touch screen, or the like.

After unsecured communication between the medical device 1 and the remote device 2 via the communications layer 5 has been established, the medical device 1 and the remote device 2 are wirelessly connected by a security and transport layer 10. Physically, the security and transport layer 10 may form a single layer as shown in Fig. 1, or it may instead be partitioned into a security layer and a separate transport layer. In either case, the asymmetric-key cryptographic part of the method of the invention and the derivation of the symmetric key take place via the security portion of the security and transport layer 10. I.e. generation of an asymmetric key pair, generation of a key request message and sending of the key request message together with the public key to the medical device, generation and encryption of a pre-master key by the medical device, generation of a key response message and sending of the key response message together with the encrypted pre-master key to the remote device, decryption of the encrypted pre-master key with the private key by the remote device, derivation of a master key, and the optional verification step are performed on the security and transport layer(s) 10 of the medical device 1 and of the remote device 2. Following successful generation of the master key a secure link (i.e. secure communications) is established between the medical device 1 and the remote device 2, and application data can be transmitted between the two devices 1 and 2 via an application layer 11. When the medical device 1 and the remote device 2 communicate wirelessly in a secure manner, the transport portion of the security and transport layer 10 may be optionally used for error recognition and retransmission of data.

The security and transport layer(s) 10 that is used for the optional verification step is preferably independent from the application layer that is used for encrypted communication by means of the master key (so-called out-of-band verification). The security and transport layer(s) 10 that is used for the verification step shall also not be susceptible to data manipulation.

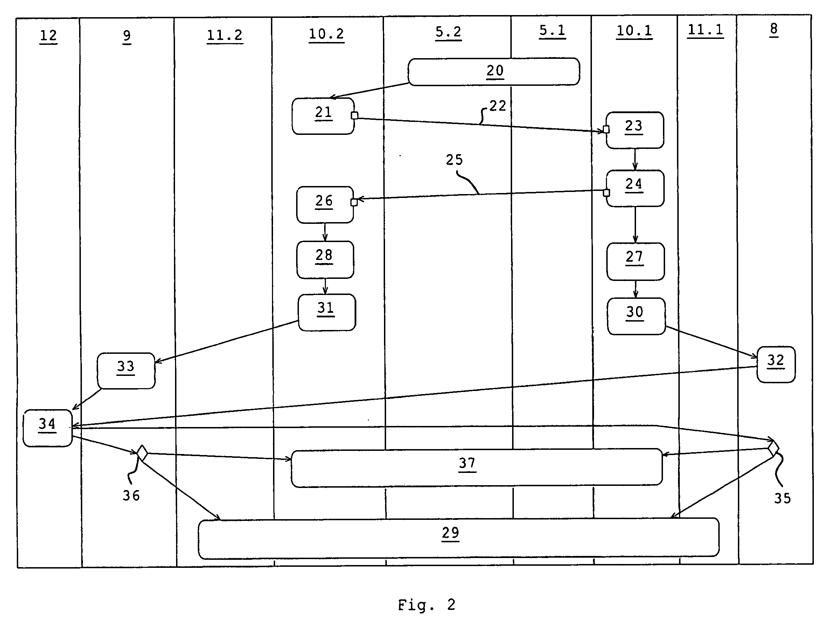

Referring to Fig. 2, a flowchart is shown of a first embodiment of the method according to the invention. The columns of Fig. 2 indicate the entities to which a certain method step or action is allocated. Column 5.1 represents the communications layer of the medical device 1. Column 5.2 represents the communications layer of the remote device 2. Column 10.1 represents the security and transport layer of the medical device 1. Column 10.2 represents the security and transport layer of the remote device 2. Column 11.1 represents the application layer of the medical device 1. Column 11.2 represents the application layer of the remote device 2. Column 8 represents the user interface of the medical device 1. Column 9 represents the user interface of the remote device 2. Column 12 represents the user who is expected to perform an action.

In a first step 20, communication between the medical device 1 and the remote device 2 is established via the communications layers 5.1, 5.2, the communication being neither authenticated nor encrypted. The amount of communication connections that the medical device 1 can accept in a certain time frame may be limited to prevent denial-of-service attacks and to prevent an attacker from gaining too much insight into the cryptographic actions performed by the medical device 1.

In step 21 the remote device 2 generates in its security and transport layer 10.2 an asymmetric RSA key pair that comprises an RSA public key and an RSA private key, each RSA key preferably containing at least 2048 bits. In step 22, the remote device 2 then generates a key request message and sends it via the security and transport layers 10.2, 10.1 to the medical device 2, thereby indicating that the remote device wishes to start the key exchange protocol. Along with the key request message the remote device 2 sends the RSA public key to the medical device, which the medical device 2 shall use for encryption. For sending, the RSA public key is preferentially PEM encoded (PEM = Privacy-enhanced Electronic Mail). The key request message preferably contains random data, in particular 28 random bytes, and a time stamp, in particular a 4 byte time stamp, to prevent replay attacks. For generation of the random bytes e.g. the so-called Blum-Blum-Shub generator may be used or the less secure, but significantly faster so-called ISAAC random number generator (ISAAC = Indirection, Shift, Accumulate, Add, and Count). The length of the public key that the medical device 1 has to accept can be restricted to avoid using computationally expensive keys.

In step 23, the medical device 1 generates a pre-master key and encrypts the pre-master key in step 24 by the RSA public key that it has received from the remote device 1. For generation of the pre-master key the medical device 1 preferably chooses a random 32 byte key in its security and transport layer 10.1. In step 25, the medical device 1 generates a key response message and answers the key request message by sending the key response message along with the encrypted pre-master key via the security and transport layers 10.1, 10.2 to the remote device 2. For sending, the encrypted pre-master key is preferentially OAEP padded (OAEP = Optimal Asymmetric Encryption Padding) or padded in accordance with another secure padding function. The key response message preferably contains random data, in particular 28 random bytes, and a time stamp, in particular a 4 byte time stamp, to prevent replay attacks. For generation of the random bytes e.g. the so-called Blum-Blum-Shub generator or the so-called ISAAC random number generator may be used.

In step 26, the remote device 2 decrypts the received encrypted pre-master key using its RSA private key in its security and transport layer 10.2. In steps 27 and 28 both the medical device 1 and the remote device 2 use the random values exchanged in the key request message and the key response message to derive a new key, namely the master key, from the pre-master key in the security and transport layers 10.1, 10.2. The master key is preferentially derived from the pre-master key by means of a pseudo-random function (PRF) whose parameters are - apart from the pre-master key - in particular the random bytes contained in the key request message and in the key response message and preferably an additional fixed string such as for example the string "master secret". The pseudo-random function may make use of one or preferably two or more different hash functions such as e.g. MD5 (Message-Digest Algorithm 5), SHA1 (SHA = Secure Hash Algorithm), RIPEMD-160 (RIPEMD = RACE Integrity Primitives Evaluation Message Digest), SHA-224, and SHA-256, with RIPEMD-160 and SHA-224 or RIPEMD-160 and SHA-256 being preferred combinations of two hash functions. The generated master key can then be used in step 29 as symmetric key within a symmetric-key cryptosystem to authenticate and encrypt application data that shall be communicated and exchanged between the medical device 1 and the remote device 2 on the application layers 11.1, 11.2.

To avoid man-in-the-middle attacks the method according to the invention preferably comprises a verification step before application data that is encrypted with the master key is actually transmitted via the application layers 11.1, 11.2 in step 29, to ensure that the messages exchanged in steps 22 and 25 between the medical device 1 and the remote device 2 (also called protocol messages) have not been manipulated by an attacker.

In steps 30 and 31 both the medical device 1 and the remote device 2 compute independently from one another verification data (also called authentication data) in the security and transport layers 10.1, 10.2 as parameters, preferably by using a pseudo-random function with the previously derived master key and the previously exchanged protocol messages, i.e. with the master key, the key request message along with the public key and the key response message along with the encrypted pre-master key as parameters. The verification data each consist exemplarily of 12 bytes, but may consist of more or less bytes, e.g. of 8 bytes, for practical purposes. In steps 32 and 33 both the medical device 1 and the remote device 2 display the verification data they each computed on their respective user interfaces 8, 9 to the user 12. In step 34, the user 12 has to compare the verification data displayed on the user interface 8 of the medical device 1 with the verification data displayed on the user interface 9 of the remote device 2. In steps 35, 36, the user 12 has then to make an appropriate user input on both user interfaces 8, 9. If the user confirms in steps 35, 36 by an appropriate user input that the displayed verification data are actually identical (i.e. if authentication is confirmed), then the method may proceed to step 29 and the cryptographically secure exchange/communication of application data via application layers 11.1, 11.2 may be started. If, however, in steps 35, 36 the user 12 does not confirm that the displayed verification data are identical (i.e. if authentication is not confirmed) or if the verification step is cancelled, then the connection establishing on the security and transport layers 10.1, 10.2 is terminated and communication between the medical device 1 and the remote device 2 on the communications layers 5.1, 5.2 is abandoned in step 37.

If the medical device 1 and the remote device 2 already exchanged cryptographic key information in form of the public key and the pre-master key and if both have computed the master key, then the devices 1 and 2 preferably may re-use the already computed master key in one or more subsequent sessions, thereby resuming the previous session, without the need to first compute a new asymmetric key pair, exchange a public key and compute and exchange a new pre-master key, i.e. without the need for negotiating new keys. To provide the option of session resumption, the key request message and preferably also the key response message each comprise a session identifier that exemplarily is 32 bits long, but can also contain more or less bits depending on boundary conditions and safety requirements.

If the remote device 2 wants to end a currently running session where the master key has already been computed and application data may already have been exchanged in step 29, then the remote device sends a key request message containing null-bits as session identifier to the medical device 1 to indicate that the remote device 2 does not wish to continue the current session (step not shown). Upon receipt of this key request message the medical device 1 chooses a random unique session identifier for the current session and sends it within the key response message to the remote device 2 (step not shown). Both the remote device 2 and the medical device 1 then store the session identifier chosen by the medical device 1 along with the master key derived in the current session in their respective memory, provided that generation of the master key and key exchange is complete (step not shown). With the session identifier and the corresponding master key being stored in the respective memory of the remote device 2 and the medical device 1, the previous session can be resumed later without the need of re-negotiating a new master key.

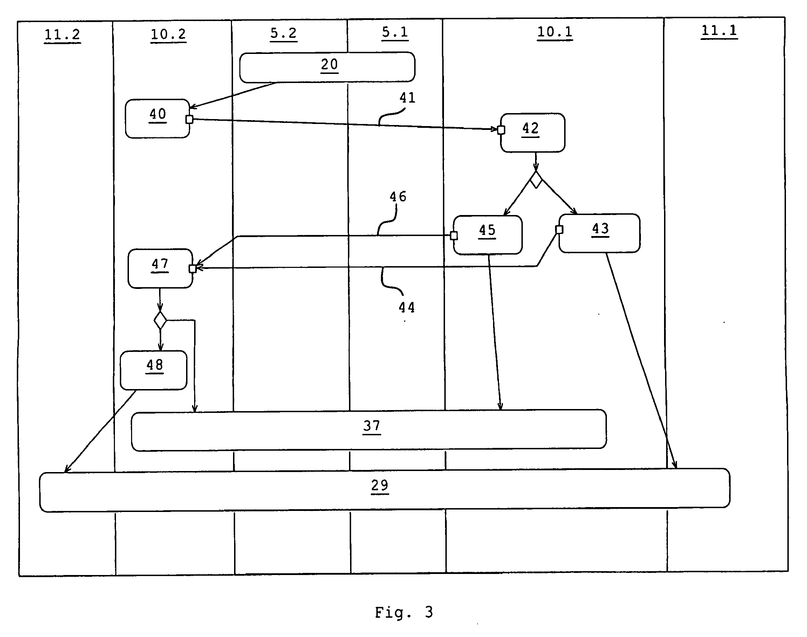

Fig. 3 depicts a flowchart of a resumption of a previous session according to the invention, the previous session having been started in accordance with the first embodiment shown in Figure 2. The verification step depicted in Fig. 2 (i.e. steps 30 to 36) is not shown but may of course also form part of the second embodiment of the invention to prevent a man-in-the-middle-attack. The meaning of the columns is as in Fig. 2. Method steps that have the same function as method steps of the embodiment shown in Fig. 2 have the same reference numerals.

As with the embodiment in Fig. 2, in step 20 unencrypted and unauthenticated communication is established between the medical device 1 and the remote device 2 via the communications layers 5.1, 5.2. If the remote device 2 wishes to resume a previous session whose session identifier and master key have been stored, it requests resuming of the previous session by generating a key request message that contains the session identifier of the session to be resumed (step 40) and sends it via the security and transport layers 10.2, 10.1 to the medical device 1 (step 41). Upon receipt of the key request message the medical device decides in step 42 whether to accept the request for session resumption or whether to decline the request. For example, to each session there may be assigned a certain validity time after which the session is considered as invalid and may not be resumed and after which a request to resume this session is declined by the medical device 1, so that a new session has to be started and established.

If the medical device 1 accepts the request for session resumption in step 43, in particular because the validity time of the previous session that shall be resumed has not expired, it generates a key response message containing the session identifier of the session to be resumed, reads the master key of the session to be resumed from its memory and sends the key response message in step 44 to the remote device 2. If the medical device 1, however, does not accept the request for session resumption in step 45, for example because the session's validity time has expired, then it generates a key response message containing only null-bits as session identifier, sends this key response message in step 46 to the remote device 2, stops the connection establishing on its security and transport layer 10.1 and disconnects/closes the connection between the medical device 1 and the remote device 2 on its communications layer 5.1 in step 37.

Upon receipt of the key response message the remote device 2 checks in step 47 the validity of the session identifier that the key response message contains. If this session identifier is invalid, i.e. contains only null-bits, the remote device 2 moves to step 37 and stops the connection establishing on its security and transport layers 10.2 and disconnects/closes the connection between the medical device 1 and the remote device 2 on its communications layer 5.2. The remote device 2 then has to start a new session with the medical device 1 as described with respect to Fig. 1 to negotiate keys and to derive at a new master key. If the session identifier of the key response message, however, corresponds to the session of which resumption has been requested, then the remote device 2 reads in step 48 the stored master key from its memory and communication and exchange of application data that is authenticated and encrypted with the master key is resumed with the medical device 1 in step 29.

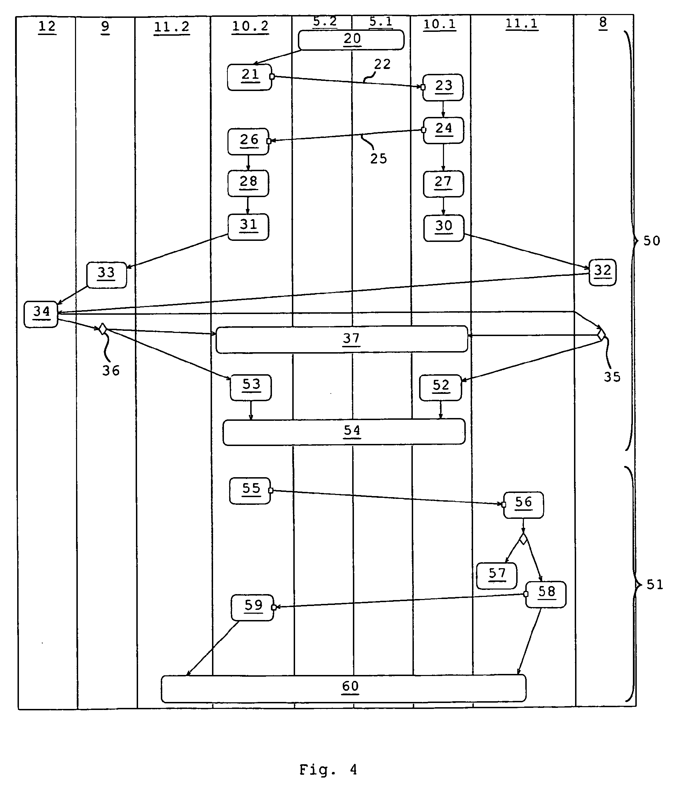

According to a further embodiment of the method of the invention the connection between the medical device 1 and the remote device 2 is preferably closed and communication between the two devices 1 and 2 is thus terminated after the derivation of the master key or, if applicable, after the performing of the verification step, i.e. after the user has confirmed that the verification data computed by the remote device 2 and by the medical device 1 have the same values. I.e. the connection between the medical device 1 and the remote device 2 is preferably closed after the pairing of the two devices 1 and 2 is complete. With this embodiment communication/connection between the medical device 1 and the remote device 2 has to be established anew after the closing of the connection after successful pairing, so that key exchange and connection establishing for application data exchange are split into two separate process parts to be performed by the remote device 2 and the medical device 1. Referring to Fig. 4, reference numeral 50 denotes a flowchart of the key exchange part and reference numeral 51 denotes a flowchart of the subsequent connection establishing part for application data exchange. The verification step depicted in Fig. 2 (i.e. steps 30 to 37) is optional and may be omitted. The meaning of the columns is as in Fig. 2. Method steps that have the same function as method steps of the embodiment shown in Fig. 2 have the same reference numerals.

The key exchange part 50 basically corresponds to the first embodiment of the method of the invention depicted in Fig. 2 apart from step 29 and it is herewith referred to the above description thereof. In the key exchange part 50, the combination of the key request message and the public key that is sent in step 22 from the remote device 2 to the medical device 1 takes preferably the form of a dedicated key exchange message in form of a key request message whose parameters are the public key (in particular an RSA public key) and a session identifier but preferably no random values. For a new session this session identifier contains only null-bits. Similarly, the combination of the key response message and the encrypted pre-master key that is sent in step 25 from the medical device 1 to the remote device 2 takes preferably the form of a dedicated key exchange message in form of a key response message whose parameters are the encrypted pre-master key and the session identifier but preferably no additional random values, the session identifier preferably being chosen by the medical device 1 to identify the current session. Regarding resumption of a previous session, combinations of the key exchange part 50 with the embodiment depicted in Fig. 3 are possible.

After the master key has been derived in steps 28 and 29 and, if a verification step is provided as depicted in Fig. 4, after the user 12 has confirmed in steps 35, 36 that the displayed verification data are identical (i.e. after authentication is confirmed), the medical device 1 and the remote device 2 store the computed master key each in their respective memory in steps 52 and 53 and it is established in step 54 that pairing of the medical device 1 and the remote device 2 is completed with the connection between the medical device 1 and the remote device 2 preferably being closed on the communications layers 5.1, 5.2, if no further actions on levels higher than the communications layers 5.1, 5.2 (e.g. on the security and transport layers 10.1, 10.2) are planned.

For re-establishing connection between the medical device 1 and the remote device 2, the remote device 2 sends in step 55 of the connection establishing part 51 a connection request, in particular a so-called SYN-message (synchronizing message), to the medical device 1 via the security and transport layers 10.1 and 10.2, the connection request containing the session identifier assigned in the key exchange part 50. In step 56, the medical device 1 checks if the session identifier is valid, in particular if the validity time of the session that the session identifier has been assigned to is not expired. If the session identifier is invalid, the medical device 1 silently discards the connection request in step 57 which the remote device 2 then notices through timeout. If the medical device 1 deems the session identifier to be valid, it accepts the connection request and sends in step 58 an acknowledgement message, in particular a so-called SYN-acknowledgement message, to the remote device 2 via the security and transport layers 10.1 and 10.2 and reads the stored master key of the identified session from its memory. The acknowledgement message has the system identifier as parameter. Up till now communication between the medical device 1 and the remote device 2 is neither authenticated nor encrypted. Upon receipt of the acknowledgement message the remote device 2 reads in step 59 the stored master key of the identified session from its memory. Following this the remote device 2 starts in step 60 with the exchange of application data with the medical device 1 via the application layers 11.1 and 11.2, the application data being authenticated and encrypted with the master key.

## Claims

1. A method for establishing cryptographic communications between a remote device (2) and a medical device (1) with the medical device (1) having less processing power than the remote device (2), comprising the steps of:
- establishing unencrypted communication between the remote device (2) and the medical device (1),
- generating an asymmetric key pair by the remote device (2), the asymmetric key pair comprising a public key and a private key,
- generating a key request message by the remote device (2) and sending the key request message together with the public key to the medical device (1),
- generating a pre-master key by the medical device (1) and encrypting the pre-master key with the received public key by the medical device (1),
- generating a key response message by the medical device (1) and sending the key response message together with the encrypted pre-master key from the medical device (1) to the remote device (2),
- decrypting the encrypted pre-master key with the private key by the remote device (2),
- deriving a master key as symmetric key from the pre-master key by using the key request message and the key response message for deriving the master key from the pre-master key by each of the medical device (1) and the remote device (2), the master key to be used for both decryption and encryption of application data to be communicated,
wherein the key request message and the key response message contain random data and a time stamp, wherein the key request message and/or the key response message contain a session identifier,
- completing pairing of the medical device (1) and the remote device (2) and terminating communication between the medical device (1) and the remote device (2),
wherein after termination of the communication, the remote device (2) sends a connection request to the medical device (1), the connection request containing the session identifier contained in the key request message and/or in the key response message.

2. The method according to claim 1, wherein the medical device (1) is an insulin pump.

3. The method according to claim 1 or 2, wherein the asymmetric key pair is an RSA key pair.

4. The method according to one of the preceding claims, wherein the remote device (2) and the medical device (1) each compute verification data using the master key, and wherein communication of the application data may first start after it has been confirmed that the verification data computed by the remote device (2) and the medical device (1) have the same values.

5. The method according to claim 4, wherein the remote device (2) and the medical device (1) each display the computed verification data to a user (12), and wherein communication of the application data may first start after the user (12) has confirmed that the verification data computed by the remote device (2) and the medical device (1) have the same values.

6. The method according to claim 4, wherein the verification data computed by one of the devices (1, 2) is transmitted to the other device (2, 1) with the other device (2, 1) performing a comparison of the verification data transmitted to it with the verification data computed by it, and wherein communication of the application data may first start after it has been confirmed through the comparison that the verification data computed by the remote device (2) and the medical device (1) have the same values.

7. The method according to claim 6, wherein the verification data computed by one of the devices (1, 2) is transmitted to the other device (2, 1) via a wireless communication interface.

8. The method according to one of the preceding claims, wherein the key request message and the key response message contain a session identifier.

9. The method according to claim 8, wherein for requesting resuming of a previous session the key request message that the remote device (2) sends to the medical device (1) contains the session identifier of the session that shall be resumed.

10. The method according to claim 9, wherein upon receipt of a key request message with a session identifier the medical device (1) has the options of accepting and of declining the request to resume the session identified by the session identifier.

11. The method according to claim 10, wherein a validity time period is defined after which a request to resume a session is declined, so that a new session has to be started.

12. The method according to claim 10 or 11, wherein if the medical device (1) declines a request to resume a session, the key response message that the medical device (1) sends to the remote device (2) contains null-bits as session identifier, and wherein after sending of the key response message the medical device (1) preferably terminates communications.

13. The method according to claim 12, wherein the remote device (2) terminates communications after the medical device (1) has declined the request to resume a session.

14. The method according to one of the claims 10 to 13, wherein the medical device (1) reads the master key of the session to be resumed from memory and sends a key response message to the remote device (2) that contains the session identifier of the session to be resumed, if the medical device (1) accepts a request to resume a session.

15. The method according to claim 14, wherein the remote device (2) reads the master key of the session to be resumed from memory if the medical device (1) accepts the request to resume a session, with the master key to be used for both decryption and encryption of the application data to be communicated.

16. The method according to one of the preceding claims, wherein after the derivation of the master key the pairing of the medical device (1) and the remote device (2) is completed and communication between the medical device (1) and the remote device (2) is terminated.

17. The method according to one of claims 1 to 16, wherein after it has been confirmed that the verification data computed by the remote device (2) and the medical device (1) have the same values the pairing of the medical device (1) and the remote device (2) is completed and communication between the medical device (1) and the remote device (2) is terminated.

18. The method according to claim 1, wherein the medical device (1) discards the connection request, if the session identifier is invalid.

19. The method according to claim 1, wherein the medical device (1) sends an acknowledgement message to the remote device (2), if it accepts the connection request, and reads the master key of the session identified by the session identifier of the connection request from memory, and wherein the remote device (2) after receipt of the acknowledgement message reads the master key from memory, so that cryptographic communication of the application data can commence.

20. A system comprising a remote device (2) and a medical device (1) with the medical device (1) having less processing power than the remote device (2), the system being designed to carry out the method according to one of the preceding claims.

21. The system according to claim 20, wherein the medical device (1) is an insulin pump.

## Patentansprüche

1. Verfahren zum Herstellen verschlüsselter Kommunikation zwischen einem externen Gerät (2) und einem Medizingerät (1), wobei das Medizingerät (1) weniger Prozessleistung als das externe Gerät (2) besitzt, umfassend die folgenden Schritte:
- Herstellen unverschlüsselter Kommunikation zwischen dem externen Gerät (2) und dem Medizingerät (1),
- Erzeugen eines asymmetrischen Schlüsselpaars durch das externe Gerät (2), wobei das asymmetrische Schlüsselpaar einen öffentlichen Schlüssel und einen privaten Schlüssel umfasst,
- Erzeugen einer Schlüssel-Anfragenachricht durch das externe Gerät (2) und Senden der Schlüssel-Anfragenachricht zusammen mit dem öffentlichen Schlüssel an das Medizingerät (1),
- Erzeugen eines Prä-Master-Schlüssels durch das Medizingerät (1) und Verschlüsseln des Prä-Master-Schlüssels mit dem empfangenen öffentlichen Schlüssel durch das Medizingerät (1),
- Erzeugen einer Schlüssel-Antwortnachricht durch das Medizingerät (1) und Senden der Schlüssel-Antwortnachricht zusammen mit dem verschlüsselten Prä-Master-Schlüssel von dem Medizingerät (1) an das externe Gerät (2),
- Entschlüsseln des verschlüsselten Prä-Master-Schlüssels mit dem privaten Schlüssel durch das externe Gerät (2),
- Ableiten eines Master-Schlüssels als symmetrischer Schlüssel von dem Prä-Master-Schlüssel unter Verwendung der Schlüssel-Anfragenachricht und der Schlüssel-Antwortnachricht zum Ableiten des Master-Schlüssels von dem Prä-Master-Schlüssel durch sowohl das Medizingerät (1) als auch das externe Gerät (2), wobei der Master-Schlüssel sowohl für die Entschlüsselung als auch die Verschlüsselung von zu kommunizierenden Anwendungsdaten zu verwenden ist, wobei die Schlüssel-Anfragenachricht und die Schlüssel-Antwortnachricht Zufallsdaten und einen Zeitstempel enthalten, wobei die Schlüssel-Anfragenachricht und/oder die Schlüssel-Antwortnachricht einen Session-Identifikator enthalten,
- Fertigstellen des Paarens des Medizingeräts (1) und des externen Geräts (2) und Beenden der Kommunikation zwischen dem Medizingerät (1) und dem externen Gerät (2),
wobei das externe Gerät (2) nach Beenden der Kommunikation eine Verbindungsanfrage an das Medizingerät (1) sendet, wobei die Verbindungsanfrage den Session-Identifikator enthält, der in der Schlüssel-Anfragenachricht und/oder in der Schlüssel-Antwortnachricht enthalten ist.

2. Verfahren nach Anspruch 1, wobei das Medizingerät (1) eine Insulinpumpe ist.

3. Verfahren nach einem der Ansprüche 1 bis 2, wobei das asymmetrische Schlüsselpaar ein RSA-Schlüsselpaar ist.

4. Verfahren nach einem der vorangehenden Ansprüche, wobei das externe Gerät (2) und das Medizingerät (1) jeweils Verifizierungsdaten unter Verwendung des Master-Schlüssels berechnen und wobei die Kommunikation der Anwendungsdaten erst starten darf, nachdem bestätigt ist, dass die Verifikationsdaten, die von dem externen Gerät (2) und dem Medizingerät (1) berechnet wurden, dieselben Werte besitzen.

5. Verfahren nach Anspruch 4, wobei jeweils das externe Gerät (2) und das Medizingerät (1) die berechneten Verifikationsdaten einem Nutzer (12) anzeigen und wobei die Kommunikation der Anwendungsdaten erst starten darf, nachdem der Nutzer (12) bestätigt hat, dass die Verifikationsdaten, die von dem externen Gerät (2) und dem Medizingerät (1) berechnet wurden, dieselben Werte besitzen.

6. Verfahren nach Anspruch 4, wobei die Verifikationsdaten, die von einem der Geräte (1, 2) berechnet wurden, an das andere Gerät (2, 1) übertragen werden, wobei das andere Gerät (2, 1) einen Vergleich der an dieses übertragenen Verifikationsdaten mit den von diesem berechneten Verifikationsdaten durchführt und wobei die Kommunikation der Anwendungsdaten erst starten darf, nachdem durch den Vergleich bestätigt ist, dass die von dem externen Gerät (2) und dem Medizingerät (1) berechneten Verifikationsdaten dieselben Werte besitzen.

7. Verfahren nach Anspruch 6, wobei die von einem der Geräte (1, 2) berechneten Verifikationsdaten über eine drahtlose Kommunikationsschnittstelle an das andere Gerät (2, 1) übertragen werden.

8. Verfahren nach einem der vorangehenden Ansprüche, wobei die Schlüssel-Anfragenachricht und die Schlüssel-Antwortnachricht einen Session-Identifikator enthalten.

9. Verfahren nach Anspruch 8, wobei zur Anfrage, eine vorige Session fortzusetzen, die Schlüssel-Anfragenachricht, die das externe Gerät (2) an das Medizingerät (1) sendet, den Session-Identifikator der fortzusetzenden Session enthält.

10. Verfahren nach Anspruch 9, wobei nach Empfangen einer Schlüssel-Anfragenachricht mit einem Session-Identifikator das Medizingerät (1) die Optionen hat, die Anfrage, die durch den Session-Identifikator identifizierte Session fortzusetzen, anzunehmen oder abzulehnen.

11. Verfahren nach Anspruch 10, wobei eine Gültigkeitszeitspanne definiert ist, nach der eine Anfrage, eine Session fortzusetzen, abgelehnt wird, so dass eine neue Session gestartet werden muss.

12. Verfahren nach einem der Ansprüche 10 bis 11, wobei, wenn das Medizingerät (1) eine Anfrage, eine Session fortzusetzen, ablehnt, die Schlüssel-Antwortnachricht, die das Medizingerät (1) an das externe Gerät (2) sendet, Null-Bits als Session-Identifikator enthält und wobei das Medizingerät (1) nach Senden der Schlüssel-Antwortnachricht bevorzugt die Kommunikation beendet.

13. Verfahren nach Anspruch 12, wobei das externe Gerät (2) die Kommunikation beendet, nachdem das Medizingerät (1) die Anfrage, eine Session fortzusetzen, abgelehnt hat.

14. Verfahren nach einem der Ansprüche 10 bis 13, wobei das Medizingerät (1) den Master-Schlüssel der fortzusetzenden Session aus dem Speicher liest und eine Schlüssel-Antwortnachricht an das externe Gerät (2) sendet, die den Session-Identifikator der fortzusetzenden Session enthält, wenn das Medizingerät (1) eine Anfrage, eine Session fortzusetzen, annimmt.

15. Verfahren nach Anspruch 14, wobei das externe Gerät (2) den Master-Schlüssel der fortzusetzenden Session aus dem Speicher liest, wenn das Medizingerät (1) die Anfrage, eine Session fortzusetzen, annimmt, wobei der Master-Schlüssel sowohl zum Entschlüsseln als auch Verschlüsseln der zu kommunizierenden Anwendungsdaten zu verwenden ist.

16. Verfahren nach einem der vorangehenden Ansprüche, wobei nach der Ableitung des Master-Schlüssels das Paaren des Medizingeräts (1) und des externen Geräts (2) abgeschlossen ist und die Kommunikation zwischen dem Medizingerät (1) und dem externen Gerät (2) beendet wird.

17. Verfahren nach einem der vorangehenden Ansprüche, wobei nach Bestätigung, dass die Verifikationsdaten, die von dem externen Gerät (2) und dem Medizingerät (1) berechnet wurden, dieselben Werte besitzen, das Paaren des Medizingeräts (1) und des externen Geräts (2) abgeschlossen ist und die Kommunikation zwischen dem Medizingerät (1) und dem externen Gerät (2) beendet wird.

18. Verfahren nach Anspruch 1, wobei das Medizingerät (1) die Verbindungsanfrage verwirft, wenn der Session-Identifikator ungültig ist.

19. Verfahren nach Anspruch 1, wobei das Medizingerät (1) eine Bestätigungsnachricht an das externe Gerät (2) sendet, wenn es die Verbindungsanfrage annimmt, und den Master-Schlüssel der durch den Session-Identifikator der Verbindungsanfrage identifizierten Session aus dem Speicher liest, und wobei das externe Gerät (2) nach Erhalten der Bestätigungsnachricht den Master-Schlüssel aus dem Speicher liest, so dass verschlüsselte Kommunikation der Anwendungsdaten beginnen kann.

20. System, umfassend ein externes Gerät (2) und ein Medizingerät (1), wobei das Medizingerät (1) weniger Prozessleistung als das externe Gerät (2) besitzt, wobei das System gestaltet ist, das Verfahren nach einem der vorangehenden Ansprüche auszuführen.

21. System nach Anspruch 20, wobei das Medizingerät (1) eine Insulinpumpe ist.

## Revendications

1. Procédé permettant d'établir des communications cryptographiques entre un dispositif distant (2) et un dispositif médical (1), le dispositif médical (1) possédant moins de puissance de traitement que le dispositif distant (2), comprenant les étapes consistant à :
- établir une communication non cryptée entre le dispositif distant (2) et le dispositif médical (1),
- générer une paire de clés asymétriques par le dispositif distant (2), la paire de clés asymétriques comprenant une clé publique et une clé privée,
- générer un message de demande de clé par le dispositif distant (2) et envoyer le message de demande de clé ensemble avec la clé publique vers le dispositif médical (1),
- générer une clé pré-maitresse par le dispositif médical (1) et encrypter la clé pré-maitresse avec la clé publique reçue par le dispositif médical (1),
- générer un message de réponse de clé par le dispositif médical (1) et envoyer le message de réponse de clé ensemble avec la clé pré-maitresse encryptée depuis le dispositif médical (1) vers le dispositif distant (2),
- décrypter la clé pré-maitresse encryptée avec la clé privée par le dispositif distant (2).
- dériver une clé maîtresse en tant que clé symétrique depuis la clé pré-maitresse en utilisant le message de demande de clé et le message de réponse de clé pour dériver la clé mairesse depuis la clé pré-maitresse par chacun du dispositif médical (1) et du dispositif distant (2), la clé maîtresse devant être utilisée pour à la fois le décryptage et le cryptage de données d'application à communiquer,
dans lequel le message de demande de clé et le message de réponse de clé contiennent des données aléatoires et un horodateur, le message de demande de clé et/ou le message de réponse de clé contenant un identifiant de session,
- compléter l'appariement du dispositif médical (1) et du dispositif distant (2) et cesser la communication entre le dispositif médical (1) et le dispositif distant (2),
dans lequel après cessation de la communication, le dispositif distant (2) envoie une demande de connexion au dispositif médical (1), la demande de connexion contenant l'identifiant de session contenu dans le message de demande de clé et/ou dans le message de réponse de clé.

2. Procédé selon la revendication 1, dans lequel le dispositif médical (1) est une pompe à insuline.

3. Procédé selon la revendication 1 ou la revendication 2, dans lequel la paire de clés asymétriques est une paire de clés RSA.

4. Procédé selon l'une quelconque des revendications précédentes, dans lequel le dispositif distant (2) et le dispositif médical (1) calculent chacun des données de vérification en utilisant la clé maitresse, et dans lequel une communication des données d'application ne peut démarrer qu'après qu'il ait été confirmé que les données de vérification calculées par le dispositif distant (2) et le dispositif médical (1) ont les mêmes valeurs.

5. Procédé selon la revendication 4, dans lequel le dispositif distant (2) et le dispositif médical (1) affichent chacun les données de vérification calculées à un utilisateur (12), et dans lequel une communication des données de vérification ne peut démarrer qu'après que l'utilisateur (12) a confirmé que les données de vérification calculées par le dispositif distant (2) et le dispositif médical (1) ont les mêmes valeurs.

6. Procédé selon la revendication 4, dans lequel les données de vérification calculées par l'un des dispositifs (1, 2) sont transmises à l'autre dispositif (2, 1) avec l'autre dispositif (2, 1) effectuant une comparaison des données de vérification transmises à lui avec les données de vérification calculées par lui, et dans lequel une communication des données d'application ne peut démarrer qu'après qu'il a été confirmé par la comparaison que les données de vérification calculées par le dispositif distant (2) et le dispositif médical (1) ont les mêmes valeurs.

7. Procédé selon la revendication 6, dans lequel les données de vérification calculées par l'un des dispositifs (1, 2) sont transmises à l'autre dispositif (2, 1) via une interface de communication sans fil.

8. Procédé selon l'une quelconque des revendications précédentes, dans lequel le message de demande de clé et le message de réponse de clé contiennent un identifiant de session.

9. Procédé selon la revendication 8, dans lequel, pour effectuer la demande de reprise d'une session précédente, le message de demande de clé que le dispositif distant (2) envoie au dispositif médical (1) contient l'identifiant de session de la session qui doit être reprise.

10. Procédé selon la revendication 9, dans lequel après réception d'un message de demande de clé avec un identifiant de session, le dispositif médical (1) a le choix d'accepter ou de décliner la demande de reprendre la session identifiée par l'identifiant de session.

11. Procédé selon la revendication 10, dans lequel une période de temps de validité est définie après qu'une demande de reprise d'une session est déclinée, de sorte qu'une nouvelle session doit être démarrée.

12. Procédé selon la revendication 10 ou la revendication 11, dans lequel si le dispositif médical (1) décline une demande de reprise d'une session, le message de réponse de clé que le dispositif médical (1) envoie au dispositif distant (2) contient des bits nuls en tant qu'identifiant de session, et dans lequel après avoir envoyé le message de réponse de clé le dispositif médical (1) cesse préférablement les communications.

13. Procédé selon la revendication 12, dans lequel le dispositif distant (2) cesse les communications après que le dispositif médical a décliné la demande pour reprendre une session.

14. Procédé selon l'une quelconque des revendications 10 à 13, dans lequel le dispositif médical (1) lit la clé maitresse de la session qui doit être reprise de mémoire et envoie un message de réponse de clé au dispositif distant (2) qui contient l'identifiant de session de la session qui doit être reprise, si le dispositif médical (1) accepte une demande de reprise d'une session.

15. Procédé selon la revendication 14, dans lequel le dispositif distant (2) lit la clé maitresse de la session qui doit reprendre de mémoire si le dispositif médical (1) accepte la demande de reprendre une session, avec la clé maitresse qui doit être utilisée à la fois pour le décryptage et le cryptage des données d'application qui doivent être communiquées.

16. Procédé selon l'une quelconque des revendications précédentes, dans lequel après la dérivation de la clé maitresse, l'appariement du dispositif médical (1) et du dispositif distant (2) est terminé et la communication entre le dispositif médical (1) et le dispositif distant (2) se termine.

17. Procédé selon l'une quelconque des revendications 1 à 16, dans lequel après qu'il ait été confirmé que les données de vérification calculées par le dispositif distant (2) et le dispositif médical (1) ont les mêmes valeurs, l'appariement du dispositif médical (1) et du dispositif distant (2) est terminé et la communication entre le dispositif médical (1) et le dispositif distant (2) se termine.

18. Procédé selon la revendication 1, dans lequel le dispositif médical (1) rejette la demande de connexion, si l'identifiant de session est invalide.

19. Procédé selon la revendication 1, dans lequel le dispositif médical (1) envoie un message de reconnaissance au dispositif distant (2), s'il accepte la demande de connexion, et lit la clé maitresse de la session identifiée par l'identifiant de session de la demande de connexion de mémoire, et dans lequel le dispositif distant (2) après réception du message de reconnaissance lit la clé maitresse de mémoire, de sorte qu'une communication cryptographique des données d'application peut commencer.

20. Système comprenant un dispositif distant (2) et un dispositif médical (1), le dispositif médical (1) ayant moins de puissance de traitement que le dispositif distant (2), le système étant conçu pour effectuer le procédé selon l'une des revendications précédentes.

21. Système selon la revendication 20, dans lequel le dispositif médical (1) est une pompe à insuline.
